# EUROPEAN PATENT APPLICATION

(11) **EP 2 839 832 A2**
(43) Date of publication of application: **25.02.2015**
(21) Application number: 14190100.9
(22) Date of filing: 16.11.2004
(51) Int. Cl.: A61K 31/4439, A61K 31/4025, A61K 31/426, A61K 31/4985, A61K 31/40, A61K 31/403, A61K 31/472, A61P 9/04, A61P 9/10, A61P 13/12

(54) **Use of dipeptidyl peptidase IV inhibitors**

(30) Priority: 17.11.2003 US 520562 P; 17.11.2003 US 520563 P; 24.02.2004 US 547191 P; 24.02.2004 US 547192 P
(62) Divisional of application: 04797932.3
(71) Applicant: Novartis AG, 4056 Basel (CH)
(72) Inventor: Pratley, Richard, Sparta, NJ New Jersey 07871 (US); Foley, James E, Sparta, NJ New Jersey 07871 (US); Hughes, Thomas, Edward, Concord, MA Massachusetts 01742 (US)
(74) Representative: Hutchison, John Robert

(57) **Abstract**

Use of a Dipeptidyl peptidase IV inhibitor (DPP-IV inhibitor), preferably (S)-1-[(3-hydroxy-1-adamantyl)amino] acetyl-2-cyano-pyrrolidine or a pharmaceutically acceptable salt thereof for the prevention, delay of progression or treatment of cardiovascular diseases or damages, renal diseases or damages, Heart Failure, or Heart Failure associated diseases.

## Description

The invention relates to the use of a Dipeptidyl peptidase IV inhibitor (DPP-IV inhibitor) or a pharmaceutically acceptable salt thereof for the treatment of cardiovascular diseases or damages, renal diseases or damages, Heart Failure, or Heart Failure associated diseases, to a method of treating warm-blooded animals including humans suffering from cardiovascular diseases or damages, renal diseases or damages, Heart Failure, or Heart Failure associated diseases, by administering to a said animal in need of such treatment an effective dose of at least one DPP-IV inhibitor or a pharmaceutically acceptable salt thereof.

The renin-angiotensin system (RAS) upregulation is a well-recognized contributor to the pathogenesis of cardiovascular disease and is one of the hormonal mechanisms involved in regulation of pressure/volume homeostasis and in expression of hypertension. The renin-angiotensin system furthermore plays a major role in the development of renal damage.

Pursuing research in this field, the applicant has surprisingly discovered that Dipeptidyl peptidase IV inhibitors (DPP-IV inhibitor) attenuate cardiovascular diseases or damages, renal diseases or damages, Heart Failure, or Heart Failure associated diseases. The DPP-IV inhibitors reduce particularly the cardiovascular thickening, the matrix deposition and cardiovascular hypertrophy and hypertrophic remodeling in animals. Secondly, the amelioration of these trophic effects was independent of changes in systemic blood pressure. The advantage of a blood pressure independent effect is that in clinical practice it is difficult to reach recommended targets. It usually requires 2-3 drugs. Also, there is no threshold for blood pressure or cardiovascular and renal diseases or damages, so a blood pressure independent protection would be highly advantageous.

Congestive heart failure (CHF), or heart failure (HF), is a term used to describe any condition in which the heart is unable to adequately pump blood throughout the body and/or unable to prevent blood from "backing up" into the lungs. These conditions cause symptoms such as shortness of breath (dyspnea), fatigue, weakness, and swelling (edema) of the legs and sometimes the abdomen.

Congestive heart failure, regardless of its etiology, is characterized by a weakness of the myocardial tissue of the left and/or right ventricles of the heart and the resulting difficulty in pumping and circulating blood to the systemic and/or pulmonary systems. Myocardial tissue weakness is typically associated with circulatory and neurohumoral changes which result in a failure to deliver sufficient blood and oxygen to peripheral tissues and organs. Some of the resulting changes include higher pulmonary and systemic pressure, lower cardiac output, higher vascular resistance and peripheral and pulmonary edema. Congestive heart failure may be further expressed as shortness of breath either on exertion, at rest or paroxysmal nocturnal dyspnea. If left untreated, congestive heart failure can lead to death.

Heart failure may be described as *systolic* or *diastolic, high-output* or *low-output, acute* or *chronic, right-sided* or *left-sided,* and *forward* or *backward.* These descriptors are often useful in a clinical setting, particularly early in the patient's course, but late in the course of chronic HF the differences between them often become blurred.

Systolic Versus Diastolic Failure: The distinction between these two forms of HF, relates to whether the principal abnormality is the inability of the ventricle to contract normally and expel sufficient blood (systolic failure) or to relax and/or fill normally (diastolic failure).

High-Output versus Low-Output Heart Failure: It is useful to classify patients with HF into those with a low cardiac output, i.e., low-output HF, and those with an elevated cardiac output, i.e., high-output HF.

Acute versus Chronic Heart Failure: The prototype of acute HF is the sudden development of a large myocardial infarction or rupture of a cardiac valve in a patient who previously was entirely well. Chronic HF is typically observed in patients with dilated cardiomyopathy or multivalvular heart disease that develops or progresses slowly. Acute HF is usually predominantly systolic, and the sudden reduction in cardiac output often results in systemic hypotension without peripheral edema. In contrast, in chronic HF, arterial pressure is ordinarily well maintained until very late in the course, but there is often accumulation of edema.

Right-Sided versus Left-Sided Heart Failure: Many of the clinical manifestations of HF result from the accumulation of excess fluid behind either one or both ventricles. This fluid usually localizes upstream to (behind) the ventricle that is initially affected.

Backward versus Forward Heart Failure: For many years a controversy has revolved around the question of the mechanism of the clinical manifestations resulting from HF. A rigid distinction between *backward* and *forward* HF (like a rigid distinction between right and left HF) is artificial, since both mechanisms appear to operate to varying extents in most patients with HF.

The treatment of HF may be divided into four components: (1) removal of the precipitating cause, (2) correction of the underlying cause, (3) prevention of deterioration of cardiac function, and (4) control of the congestive HF state.

Conventionally, HF has been treated with a wide variety of drugs, including alpha-adrenergic agonists, beta-adrenergic antagonists, calcium channel antagonists, cardiac glycosides, diuretics, nitrates, phosphodiesterase inhibitors, prazosin, and a variety of vasodilators.

All of these drugs, however, have undesirable side-effects. For example, use of alpha-adrenergic agonists results in edema of the peripheral tissues. The prolonged use of *β-*adrenergic agents leads to the progressive development of desensitization to the drug. Cardiac glycosides produce toxic side- effects in the CNS, and also in the gastrointestinal and respiratory systems. They additionally produce pro-arrhythmic effects. Treatment with diuretics may result in a variety of adverse-effects, such as hyponatremia, hypokalemia, and hyperchloremic metabolic alkalosis.

Prolonged use of calcium channel antagonists, such as verapamil, diltiazem and nifedipine. render them ineffective. Moreover, calcium channel antagonists have been shown to increase the mortality rates in patients thus treated, because such compounds act to increase oxygen consumption, which further stresses the compromised heart.

Thus, there is a continued demand for new, non toxic, compounds for treating HF, improving left ventricle function, without increasing the myocardial oxygen requirement. It is also preferred that the drugs do not act directly to stimulate cardiac contractility, or produce side-effects such as changes in blood pressure and/or heart rate, since they are associated with increased mortality in patients with HF.

Pursuing research in this field, the applicant has surprisingly discovered that DPP-IV inhibitors are useful for the prevention, delay of progression or the treatment of heart failure (HF) or heart failure associated diseases.

The present invention thus concerns the use of a Dipeptidyl peptidase IV inhibitor (DPP-IV inhibitor) or pharmaceutically acceptable salt thereof, for the manufacture of a medicament for treating cardiovascular diseases or damages, renal diseases or damages, Heart Failure, or Heart Failure associated diseases.

The term "DPP-IV inhibitor" is intended to indicate a molecule that exhibits inhibition of the enzymatic activity of DPP-IV and functionally related enzymes, such as from 1-100% inhibition, and specially preserves the action of substrate molecules, including but not limited to glucagon-like peptide-1, gastric inhibitory polypeptide, peptide histidine methionine, substance P, neuropeptide Y, and other molecules typically containing alanine or proline residues in the second aminoterminal position. Treatment with DPP-IV inhibitors prolongs the duration of action of peptide substrates and increases levels of their intact, undegraded forms leading to a spectrum of biological activities relevant to the disclosed invention.

DPP-IV can be used in the control of glucose metabolism because its substrates include the insulinotropic hormones Glucagon like peptide-1 (GLP-1) and Gastric inhibitory peptide (GIP). GLP-1 and GIP are active only in their intact forms; removal of their two N-terminal amino acids inactivates them. In vivo administration of synthetic inhibitors of DPP-IV prevents N- terminal degradation of GLP-1 and GIP, resulting in higher plasma concentrations of these hormones, increased insulin secretion and, therefore, improved glucose tolerance. For that purpose, chemical compounds are tested for their ability to inhibit the enzyme activity of purified CD26/DPP-IV. Briefly, the activity of CD26/DPP-IV is measured in vitro by its ability to cleave the synthetic substrate Gly-Pro-p-nitroanilide (Gly-Pro-pNA). Cleavage of Gly-Pro-pNA by DPP-IV liberates the product p-nitroanilide (pNA), whose rate of appearance is directly proportional to the enzyme activity. Inhibition of the enzyme activity by specific enzyme inhibitors slows down the generation of pNA. Stronger interaction between an inhibitor and the enzyme results in a slower rate of generation of pNA. Thus, the degree of inhibition of the rate of accumulation of pNA is a direct measure of the strength of enzyme inhibition. The accumulation of pNA is measured with a spectrophotometer. The inhibition constant, Ki, for each compound is determined by incubating fixed amounts of enzyme with several different concentrations of inhibitor and substrate.

In the present context "a DPP-IV inhibitor" is also intended to comprise active metabolites and prodrugs thereof, such as active metabolites and prodrugs of DPP-IV inhibitors. A "metabolite" is an active derivative of a DPP-IV inhibitor produced when the DPP-IV inhibitor is metabolised. A "prodrug" is a compound that is either metabolised to a DPP-IV inhibitor or is metabolised to the same metabolite(s) as a DPP-IV inhibitor.

DPP-IV inhibitors are known in the art. For example, DPP-IV inhibitors are in each case generically and specifically disclosed e.g. in WO 98/19998,DE19616 486 A1, WO 00/34241, WO 95/15309, WO 01/72290, WO01/52825, WO 9310127, WO 9925719, WO 9938501, WO 9946272, WO 9967278 and WO 9967279.

Preferred DPP-IV inhibitors are described in the following patent applications; WO 02053548 especially compounds 1001 to 1293 and examples 1 to 124, WO 02067918 especially compounds 1000 to 1278 and 2001 to 2159, WO 02066627 especially the described examples, WO 02/068420 especially all the compounds specifically listed in the examples I to LXIII and the described corresponding analogues, even preferred compounds are 2(28), 2(88), 2(119), 2(136) described in the table reporting IC50, WO 02083128 especially examples 1 to 13, US 2003096846 especially the specifically described compounds, WO 2004/037181 especially examples 1 to 33, WO 0168603 especially compounds of examples 1 to 109, EP1258480 especially compounds of examples 1 to 60, WO 0181337 especially examples 1 to 118, WO 02083109 especially examples 1A to 1D, WO 030003250 especially compounds of examples 1 to 166, most preferably 1 to 8, WO 03035067 especially the compounds described in the examples, WO 03/035057 especially the compounds described in the examples, US2003216450 especially examples 1 to 450, WO 99/46272 especially compounds of claims 12, 14, 15 and 17, WO 0197808 especially compounds of claim 2, WO 03002553 especially compounds of examples 1 to 33, WO 01/34594 especially the compounds described in the examples 1 to 4, WO 02051836 especially examples 1 to 712, EP1245568 especially examples 1 to 7, EP1258476 especially examples 1 to 32, US 2003087950 especially the described examples, WO 02/076450 especially examples 1 to 128, WO 03000180 especially examples 1 to 162, WO 03000181 especially examples 1 to 66, WO 03004498 especially examples 1 to 33, WO 0302942 especially examples 1 to 68, US 6482844 especially the described examples, WO 0155105 especially the compounds listed in the examples 1 and 2, WO 0202560 especially examples 1 to 166, WO 03004496 especially examples 1 to 103, WO 03/024965 especially examples 1 to 54, WO 0303727 especially examples 1 to 209, WO 0368757 especially examples 1 to 88, WO 03074500 especially examples 1 to 72, examples 4.1 to 4.23, examples 5.1 to 5.10, examples 6.1 to 6.30, examples 7.1 to 7.23, examples 8.1 to 8.10, examples 9.1 to 9.30, WO 02038541 especially examples 1 to 53, WO 02062764 especially examples 1 to 293, preferably the compound of example 95 (2-{{3-(Aminomethyl)-4-butoxy-2-neopentyl-1-oxo-1,2 dihydro-6-isoquinolinyl}oxy}acetamide hydrochloride), WO 02308090 especially examples 1-1 to 1-109, examples 2-1 to 2-9, example 3, examples 4-1 to 4-19, examples 5-1 to 5-39, examples 6-1 to 6-4, examples 7-1 to 7-10, examples 8-1 to 8-8, examples 7-1 to 7-7 of page 90, examples 8-1 to 8-59 of pages 91 to 95, examples 9-1 to 9-33, examples 10-1 to 10-20, US 2003225102 especially compounds 1 to 115, compounds of examples 1 to 121,preferably compounds a) to z), aa) to az), ba) to bz), ca) to cz) and da) to dk), WO 0214271 especially examples 1 to 320 and US 2003096857, WO 2004/052850 especially the specifically described compounds such as examples 1 to 42 and compounds of claim 1, DE 102 56 264 A1 especially the described compounds such as examples 1 to 181 and the compounds of claim 5, WO 04/076433 especially the compounds specifically described, such as listed in table A, preferably the compounds listed in table B, preferably compounds I to XXXXVII, or compounds of claims 6 to 49, WO 04/071454 especially the specifically described compounds e.g. compounds 1 to 53 or compounds of tables la to If , or compounds of claims 2 to 55, WO 02/068420 especially the compounds specifically described, such as the compounds I to LXIII or Beispiele I and analogues 1 to 140 or Beispiele 2 and analogues 1 to 174 or Beispiele 3 and analogues 1, or Beispiele 4 to 5, or Beispiele 6 and analogues 1 to 5, or Beispiele 7 and analogues 1-3, or Beispiele 8 and analogue 1, or Beispiele 9, or Beispiele 10 and analogues 1 to 531 even preferred are compounds of claim 13, WO 03/000250 especially the compounds specifically described, such as the compounds 1 to 166, preferably compounds of examples 1 to 9, WO 03/024942 especially the compounds specifically described, such compounds 1 to 59, compounds of table 1 (1 to 68), compounds of claims 6, 7, 8, 9, WO 03024965024942 especially the compounds specifically described, such compounds 1 to 54, Wo03002593 especially the compounds specifically described, such compounds table 1 or of claims 2 to 15, WO03037327 especially the compounds specifically described, such compounds of examples 1 to 209 WO 03/000250 especially the compounds specifically described, such as the compounds 1 to 166, preferably compounds of examples 1 to 9, WO 03/024942 especially the compounds specifically described, such compounds 1 to 59, compounds of table 1 (1 to 68), compounds of claims 6, 7, 8, 9, WO 03024965024942 especially the compounds specifically described, such compounds 1 to 54, Wo03002593 especially the compounds specifically described, such compounds table 1 or of claims 2 to 15, WO03037327 especially the compounds specifically described, such compounds of examples 1 to 209, WO0238541, WO0230890.

WO 03/000250 especially the compounds specifically described, such as the compounds 1 to 166, preferably compounds of examples 1 to 9, WO 03/024942 especially the compounds specifically described, such compounds 1 to 59, compounds of table 1 (1 to 68), compounds of claims 6, 7, 8, 9, WO 03024965 especially the compounds specifically described, such compounds 1 to 54, Wo03002593 especially the compounds specifically described, such compounds table 1 or of claims 2 to 15, WO03037327 especially the compounds specifically described, such compounds of examples 1 to 209, WO0238541 especially the compounds specifically described, such compounds of examples 1 to 53, WO 03/002531 especially the compounds specifically described preferably the compounds listed on page 9 to 13, most preferably the compounds of examples 1 to 46 and even preferred compound of example 9, U.S. Patent No. 6,395,767 preferably compound of examples 1 to 109 most preferably compound of example 60.

In each case in particular in the compound claims and the final products of the working examples, the subject matter of the final products, the pharmaceutical preparations and the claims are hereby incorporated into the present application by reference to these publications.

WO 9819998 discloses N- (N'-substituted glycyl)-2-cyano pyrrolidines, in particular 1-[2-[5-Cyanopyridin-2-yl] amino]- ethylamino] acetyl-2-cyano- (S)- pyrrolidine.

Preferred compounds described in WO03/002553 are listed on pages 9 to 11 and are incorporated into the present application by reference.

DE19616 486 A1 discloses val-pyr, val-thiazolidide, isoleucyl-thiazolidide, isoleucyl-pyrrolidide, and fumar salts of isoleucyl-thiazolidide and isoleucyl-pyrrolidide.

Published patent application WO 0034241 and published patent US 6110949 disclose N-substituted adamantyl-amino-acetyl-2-cyano pyrrolidines and N-(substituted glycyl)-4-cyano pyrrolidines respectively. DPP-IV inhibitors of interest are specially those cited in claims 1 to 4. In particular these applications describe the compound 1-[[(3-Hydroxy-1-adamantyl) amino]acetyl]-2-cyano-(S)-pyrrolidine (also known as LAF237).

WO 9515309 discloses amino acid 2- cyanopyrrolidine amides as inhibitors of DPP-IV and WO 9529691 discloses peptidyl derivates of diesters of alpha-aminoalkylphosphonic acids, particularly those with proline or related structures. DPP-IV inhibitors of interest are specially those cited in Table 1 to 8.

In WO 01/72290 DPP-IV inhibitors of interest are specially those cited in example 1 and claims 1, 4,and 6.

WO01/52825 specially discloses (S)-1 -{2-[5-cyanopyridin-2yl)amino]ethyl-aminoacetyl)-2-cyano- pyrrolidine or (S)-1 -[(3-hydroxy-1 adamantyl)amino]acetyl-2-cyano-pyrrolidine (LAF237).

WO 9310127 discloses proline boronic esters useful as DPP-IV inhibitors. DPP-IV inhibitors of interest are specially those cited in examples 1 to 19.

Published patent application WO 9925719 discloses sulphostin, a DPP-IV inhibitor prepared by culturing a Streptomyces microorganism.

WO 9938501 discloses N-substituted 4- to 8-membered heterocyclic rings. DPP-IV inhibitors of interest are specially those cited in claims 15 to 20.

WO 9946272 discloses phosphoric compounds as inhibitors of DPP-IV. DPP-IV inhibitors of interest are specially those cited in claims 1 to 23.

Other preferred DPP-IV inhibitors are the compounds of formula I, II or III disclosed in the patent application WO 03/057200 on page 14 to 27. Most preferred DPP-IV inhibitors are the compounds specifically described on pages 28 and 29.

Published patent applications WO 9967278 and WO 9967279 disclose DPP-IV prodrugs and inhibitors of the form A-B-C where C is either a stable or unstable inhibitor of DPP-IV.

Preferably, the N-peptidyl-O-aroyl hydroxylamine is a compound of formula VII 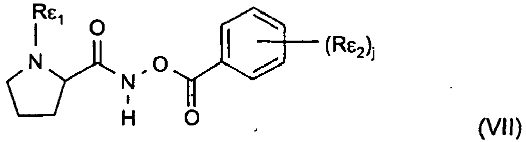 wherein
j is 0, 1 or 2;
Rε₁ represents the side chain of a natural amino acid; and
Rε₂ represents lower alkoxy, lower alkyl, halogen or nitro;
or a pharmaceutically acceptable salt thereof.

In a very preferred embodiment of the invention, the N-peptidyl-O-aroyl hydroxylamine is a compound of formula VIIa 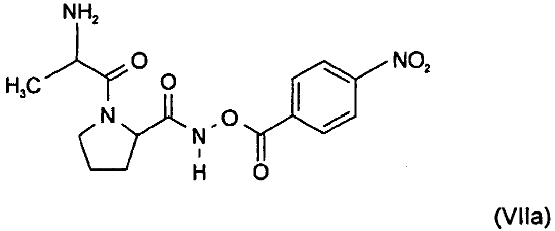 or a pharmaceutically acceptable salt thereof.

N-Peptidyl-O-aroyl hydroxylamines, e.g. of formula VII or VIIa, and their preparation are described by H.U. Demuth et al. in J. Enzyme Inhibition 1988, Vol. 2, pages 129-142, especially on pages 130-132.

Preferred DPP-IV inhibitors are those described by Mona Patel and col. (Expert Opinion Investig Drugs. 2003 Apr;12(4):623-33) on the paragraph 5, especially P32/98, K-364, FE-999011, BDPX, NVP-DDP-728 and others, which publication is hereby incorporated by reference especially the described DPP-IV inhibitors.

FE-999011 is described in the patent application WO 95/15309 page 14, as compound No. 18.

Another preferred inhibitor is the compound BMS-477118 disclosed in U.S. Patent No. 6,395,767 (compound of example 60) also known as is (1S,3S,5S)-2-[(2S)-2-amino-2-(3-hydroxytricyclo[3.3.1.1^{3,7}]dec-1-yl)-1-oxoethyl]-2-azabicyclo[3.1.0]hexane-3-carbonitrile, benzoate (1:1) as depicted in Formula M of the patent application WO 2004/052850 on page 2, and the corresponding free base, (IS,3S,5S)-2-[(2S)-2-amino-2- (3-hydroxy-tricyclo[3.3.1.1^{3,7}]dec-1-yl)-1-oxoethyl]-2-azabicyclo-[3.1.0]hexane-3-carbonitrile (M') and its monohydrate (M") as depicted in Formula M of the patent application WO 2004/052850 on page 3.

Another preferred inhibitor is the compound GSK23A disclosed in WO 03/002531 (example 9) also known as (2S,4S)-1- ((2R)-2-Amino-3-[(4-methoxybenzyl)sulfonyl]-3-methylbutanoyl)-4-fluoropyrrolidine-2-carbonitrile hydrochloride.

Other very preferred DPP-IV inhibitors of the invention are described in the International patent application WO 02/076450 (especially the examples 1 to 128) and by Wallace T. Ashton (Bioorganic & Medicinal Chemistry Letters 14 (2004) 859-863) especially the compound 1 and the compounds listed in the tables 1 and 2. The preferred compound is the compound 21 e (table 1) of formula

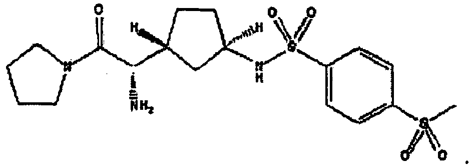

P32/98 or P3298 (CAS number: 251572-86-8) also known as 3-[(2S,3S)-2-amino-3-methyl-1-oxopentyl]thiazolidine can be used as 3-[(2S,3S)-2-amino-3-methyl-1-oxopentyl]thiazolidine and (2E)-2-butenedioate (2:1) mixture such as shown below 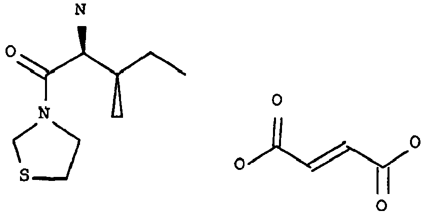 and is described in WO 99/61431 in the name of Probiodrug and also the compound P 93/01.

Other preferred DPP-IV inhibitors are the compounds disclosed in the patent application WO 02/083128 such as in the claims 1 to 5. Most preferred DPP-IV inhibitors are the compounds specifically described by the examples 1 to 13 and the claims 6 to 10.

Other preferred DPP-IV inhibitors are described in the patent applications WO 2004/037169 especially those described in the examples 1 to 48 and WO 02/062764 especially the described examples 1 to 293, even preferred are the compounds 3-(aminomethyl)-2-isobuthyl-1-oxo-4-phenyl-1,2-dihydro-6-isoquinolinecarboxamide and 2-{[3-(aminomethyl)-2-isobuthyl-4-phenyl-1-oxo-1,2-dihydro-6-isoquinolyl]oxy}acetamide described on page 7 and also in the patent application WO2004/024184 especially in the reference examples 1 to 4.

Other preferred DPP-IV inhibitors are described in the patent application WO 03/004498 especially examples 1 to 33 and most preferably the compound of the formula 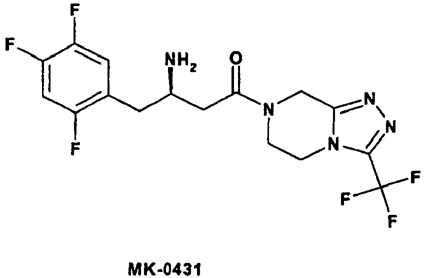 described by the example 7 and also known as MK-0431.

Preferred DPP-IV inhibitors are also described in the patent application WO 2004/037181 especially examples 1 to 33, most preferably the compounds described in the claims 3 to 5.

Preferred DPP-IV inhibitors are N-substituted adamantyl-amino- acetyl-2-cyano pyrrolidines, N (substituted glycyl)-4-cyano pyrrolidines, N- (N'-substituted glycyl)-2-cyanopyrrolidines, N-aminoacyl thiazolidines, N-aminoacyl pyrrolidines, L-allo-isoleucyl thiazolidine, L-threo-isoleucyl pyrrolidine, and L-allo-isoleucyl pyrrolidine, 1-[2-[(5-cyanopyridin-2-yl) amino] ethylamino] acetyl-2-cyano- (S)-pyrrolidine and pharmaceutical salts thereof.

Especially preferred are 1-{2-[(5-cyanopyridin-2-yl) amino] ethylamino} acetyl-2 (S)- cyano-pyrrolidine dihydrochloride (DPP728), of formula 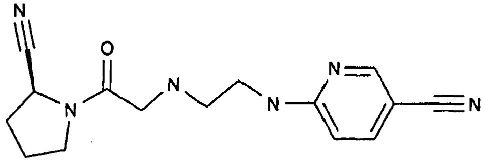 especially the dihydrochloride thereof,
and (S)-1-[(3-hydroxy-1-adamantyl)amino]acetyl-2-cyano-pyrrolidine (LAF237) of formula 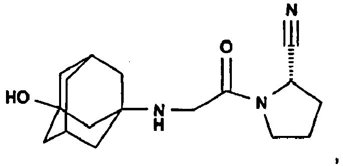 and L-threo-isoleucyl thiazolidine (compound code according to Probiodrug: P32/98 as described above), MK-0431, GSK23A, BMS-477118, 3-(aminomethyl)-2-isobuthyl-1-oxo-4-phenyl-1,2-dihydro-6-isoquinolinecarboxamide and 2-{[3-(aminomethyl)-2-isobuthyl-4-phenyl-1-oxo-1,2-dihydro-6-isoquinolyl]oxy}acetamide and optionally in any case pharmaceutical salts thereof.

DPP728 and LAF237 are the very preferred compounds and are specifically disclosed in Example 3 of WO 98/19998 and Example 1 of WO 00/34241, respectively. The DPP-IV inhibitor P32/98 (see above) is specifically described in Diabetes 1998, 47, 1253-1258. DPP728 and LAF237 can be formulated as described on page 20 of WO 98/19998 or in WO 00/34241. The preferred formulations for the administration of LAF237 are described in the US provisional application No. 60/604274.

Especially preferred are orally active DPP-IV inhibitors. In a further embodiment, preferred DPP-IV inhibitors are preferably not dipeptidic compounds and derivatives.

Any of the substances disclosed in the above mentioned patent documents, hereby included by reference, are considered potentially useful as DPP-IV inhibitors to be used in carrying out the present invention.

DPP-IV inhibitor to be used alone according to the present invention can be used in association with a carrier.

A carrier in the instant context is a tool (natural, synthetic, peptidic, non-peptidic) for example a protein which transports specific substances through the cell membrane in which it is embedded and into the cell. Different carriers (natural, synthetic, peptidic, non-peptidic) are required to transport different substances, as each one is designed to recognize only one substance, or group of similar substances.

Any means of detection known by the person skilled in the art can be used to detect the association of the DPP-IV with a carrier, for example, by labelling the carrier.

The DPP-IV inhibitor can be a peptidic or, preferably, non-peptidic one.

Most preferred are orally active DPP-IV inhibitors and pharmaceutical salts thereof.

The active ingredients or pharmaceutically acceptable salts thereof according to the present invention may also be used in form of a solvate, such as a hydrate or including other solvents, used for crystallization.

It has now been surprisingly found that DPP-IV inhibitors are useful for the prevention, delay of progression or the treatment of cardiovascular diseases or damages, renal diseases or damages, Heart Failure, or Heart Failure associated diseases.

The present invention thus concerns the use of a DPP-IV inhibitor or pharmaceutically acceptable salt thereof, for the manufacture of a medicament for the prevention, delay of progression or the treatment of cardiovascular diseases or damages, renal diseases or damages, Heart Failure, or Heart Failure associated diseases.

In particular, the present invention relates to a new use of (S)-1-[(3-hydroxy-1-adamantyl) amino] acetyl-2-cyano-pyrrolidine (LAF237) of formula (I) 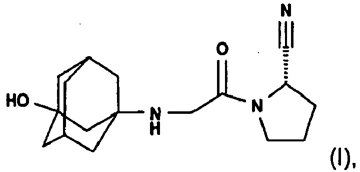 or pharmaceutically acceptable salt thereof, for the manufacture of a medicament for the prevention, delay of progression or the treatment of cardiovascular diseases or damages, renal diseases or damages, Heart Failure, or Heart Failure associated diseases.

The present invention relates furthermore to a method for the prevention, delay of progression or treatment of cardiovascular diseases or damages, renal diseases or damages, Heart Failure, or Heart Failure associated diseases, comprising administering to a warm-blooded animal, including man, in need thereof, a therapeutically effective amount of a DPP-IV inhibitor preferably (S)-1-[(3-hydroxy-1-adamantyl) amino] acetyl-2-cyano-pyrrolidine (LAF237) of formula (I).

In a further embodiment, the present invention relates to a pharmaceutical composition comprising a therapeutically effective amount of a DPP-IV inhibitor in combination with one or more pharmaceutically acceptable carriers for the treatment of cardiovascular diseases or damages, renal diseases or damages, Heart Failure, or Heart Failure associated diseases.

Furthermore it has surprisingly been discovered that the compounds disclosed herein do not have the disadvantages of the currently used compounds. It has been found that the compounds of the invention exert their beneficial effect as a consequence of having partial fatty acid oxidation (pFox) inhibiting properties and glucose oxidation activating properties, and that they are valuable for the treatment of cardiovascular diseases or damages, renal diseases or damages, Heart Failure, or Heart Failure associated diseases.

In particular, the compounds of the invention switch substrate use in the heart from fatty acids to glucose/lactates, thus improving left ventricle function, while not producing adverse side-effects such as changes in blood pressure and/or heart rate, and do not act directly to stimulate cardiac contractility, all of which would be expected from a calcium entry blocker.

It has also been surprisingly found that DPP-IV inhibitors are particularly able to slow or halt progression of myocardial remodeling, to improve endothelial function, to improve endothelial-dependent vasodilator function, to reduce progression of atherosclerosis, to markedly improve endothelial-dependent vasodilator function to acetylcholine, improvement in vasodilatory capacity, known to be severely impaired in CHF patients, decrease levels of apoptosis and necrosis.

It has been surprisingly found that DPP-IV inhibitors improve left ventricle function, without increasing the myocardial oxygen requirement. Furthermore DPP-IV inhibitors do not act directly to stimulate cardiac contractility, or produce side-effects such as changes in blood pressure and/or heart rate, since they are associated with increased mortality in patients with HF.

It has also been surprisingly found that DPP-IV inhibitors are particularly safe (non toxic) and useful for long-term administration e.g. less side effects, good absorbability into the body upon oral administration and long-lasting action.

The method or use of the present invention comprises the long-term administration to a patient of a daily dose of DPP-IV inhibitor effective to treat HF.

Most preferably, according to the present invention the cardiovascular diseases or damages are selected from cardiac hypertrophy, cardiac remodeling after myocardial infarction, pulmonary congestion and cardiac fibrosis in dilated or in hypertrophic cardiomyopathy, cardiomyopathy such as dilated cardiomyopathy or hypertrophic cardiomyopathy or diabetic cardiomyopathy, left or right ventricular hypertrophy, diabetic myopathy, stroke prevention in congestive heart failure, hypertrophic medial thickening in arteries and/or in large vessels, mesenteric vasculature hypertrophy, or artherosclerosis.

Other possible cardiovascular and renal diseases or damages are selected from diabetic macular edema, diabetic neuropathy, diabetic retinopathy, myocardial infarction, peripheral arterial disease, endothelial dysfunction with pro-inflammatory and pro-oxidant states, endothelial dysfunction, restenosis, limb ischemia, nephropathy, stroke, diabetic nephropathy or coronary heart disease.

In a further preferred embodiment the prevention, delay of progression or treatment of cardiovascular diseases or damages relates to the use of DPP-IV inhibitors to improve left ventricle function.

Most preferably, according to the present invention the renal diseases or damages are selected from renal hyperfiltration such as after portal renal ablation, proteinuria in chronic renal disease, renal arteriopathy as a consequence of hypertension, Nephrosclerosis or hypertensive nephrosclerosis, mesanglial hypertrophy.

In a further embodiment the vascular disease or damage is blood vessels injury in the kidneys most preferably in an animal or human suffering from hypertension or diabetes.

In a further embodiment the vascular disease or damage is selected from hypertrophic medial thickening in arteries and/or in large vessels, mesenteric vasculature hypertrophy or artherosclerosis, most preferably in an animal or human suffering from hypertension or diabetes.

In a further embodiment the vascular diseases or damages are selected from artherosclerosis, preferably coronary, cerebral and peripheral atherosclerosis, most preferably in an animal or human suffering from hypertension or diabetes.

In a further preferred embodiment the cardiovascular and renal diseases or damages are selected from cardiac hypertrophy such as left ventricular hypertrophy, pulmonary congestion or cardiomyopathy such as dilated cardiomyopathy or hypertrophic cardiomyopathy or diabetic cardiomyopathy, or mesanglial hypertrophy.

In a further preferred embodiment, cardiovascular diseases or damages, relate to diseases involving hypertrophy or hypertrophic remodeling in the cardiovascular system and/or in the kidney. Preferably the diseases involving hypertrophy or hypertrophic remodeling in the cardiovascular system are selected from hypertrophic cardiomyopathy, left ventricular hypertrophy or hypertrophic medial thickening in arteries and/or in large vessels.

In a further embodiment the cardiovascular diseases or damages, renal diseases or damages, Heart Failure, or Heart Failure associated diseases are preferably selected from hypertension induced cardiovascular diseases or damages, renal diseases or damages, Heart Failure, or Heart Failure associated diseases.

In a further embodiment the cardiovascular diseases or damages, renal diseases or damages, Heart Failure, or Heart Failure associated diseases are preferably selected from diabetes induced cardiovascular diseases or damages, renal diseases or damages, Heart Failure, or Heart Failure associated diseases.

The term "heart failure associated diseases" as used herein, means treating any one or more of the conditions underlying HF, including, without limitation, decreased cardiac contractility, decreased ventricular pressure such as left ventricular systolic pressure, abnormal diastolic compliance, reduced stroke volume, infection of the heart valves and/or muscle itself (endocarditis and/or myocarditis) and decreased cardiac output, while minimizing or attenuating deleterious effects commonly associated with the long-term administration of other prior art compounds, such as oxygen-wasting effects, arrhythmias, and exacerbation of angina pectoris. As further used herein, "oxygen-wasting effects" include, without limitation, symptoms and signs of congestion due to increased ventricular filling pressures, and fatigue associated with low cardiac output.
As used herein, "long-term administration" means administration for at least three weeks.

The term "cardiovascular diseases or damages" as used herein means a cardiac and/or vascular disease or a cardiac and/or vascular damage.

The term "endothelial dysfunction with pro-inflammatory and pro-oxidant states" as used herein means but is not restricted to chronic damage to the endothelium where Ang II is deeply involved.

The term "hypertension induced cardiovascular diseases or damages, renal diseases or damages, Heart Failure, or Heart Failure associated diseases" as used herein means cardiovascular diseases or damages, renal diseases or damages, Heart Failure, or Heart Failure associated diseases in animals or humans suffering from hypertension. Hypertension induced cardiovascular diseases or damages, renal diseases or damages, Heart Failure, or Heart Failure associated diseases are preferably vascular damages to the ocular, renal, cardiac, general circulation and cerebral systems.

The term "diabetes induced cardiovascular diseases or damages, renal diseases or damages, Heart Failure, or Heart Failure associated diseases" as used herein means cardiovascular or renal diseases or damages in animals or humans suffering from diabetes e.g. type 2 diabetes, such as especially artherosclerosis, hypertrophic cardiomyopathy, stroke prevention in congestive heart failure, heart failure, left ventricular hypertrophy or vascular damages to the ocular, renal, cardiac, general circulation and cerebral systems such as preferably artherosclerosis.

Cardiac, vascular or kidney hypertrophy or hypertrophic remodeling is characterized by an increase in mass of the heart, arteries, large vessels or kidney.

Hypertension induced cardiovascular diseases or damages, renal diseases or damages, Heart Failure, or Heart Failure associated diseases. Hypertension, a condition of elevated blood pressure, affects a substantial number of the human population. Consequences of persistent hypertension include vascular damage to the ocular, renal, cardiac and cerebral systems, and the risk of these complications increases as blood pressure increases. Injuries in relation to hypertension, according to the invention are preferably but not limited to cardiac hypertrophy such as right or left ventricular hypertrophy (LVH) in hypertensive patients, renal arteriopathy, and vascular diseases.

Endothelial dysfunction, is an early stage disease characterized by impaired vasomotion/tone, prothrombotic state, pro-inflammatory state and/or proliferation in arterial walls. The development of endothelial dysfunction is also speculated to be one of the several factors which might contribute to the pathogenesis of atherosclerosis and other cardiovascular diseases.

Cardiac hypertrophy or cardiac myocyte hypertrophy is an adaptive process of the heart to reduce wall stress when an increased pressure load is imposed on the myocardium. Activation of the renin-angiotensin system has been postulated to play a major role in the pathophysiology of cardiac hypertrophy and failure. In vivo studies have demonstrated that angiotensin II increases left ventricular mass and contributes to cardiac phenotype modulation independently from its effect on arterial pressure. By in vitro studies, it has been shown that angiotensin II causes cardiac myocyte hypertrophy and modulates interstitial fibrosis.

Adverse cardiac remodeling after myocardial infarction: The loss of contractile tissue by myocardial infarction (MI) is in part compensated for by hypertrophy of the remaining surviving myocardium. Larger transmural infarcts tend to expand and the whole ventricle reacts by dilatation. Adaptation to increased load of residual myocardium includes strengthening of the myocardium by increased collagen deposition. The whole process is termed remodeling of the heart (cardiac remodelling) and appears to be controlled by mechanical and humoral factors. Importantly, remodeling includes gross morphologic, histologic, cellular, and molecular changes of the infarct and scar as well as of residual myocardium. Progressive dilatation is accompanied by progressive left ventricular (LV) dysfunction and electrical instability of the heart. Patients after acute MI are at risk of recurrent ischemia, arrhythmias, and remodeling even if they are asymptomatic. In the assessment of post-MI patients, it is important to identify the patient at risk of remodeling and to apply secondary preventive therapy that includes interruption or delay of the remodeling process.

Pulmonary congestion and cardiac fibrosis in dilated or in hypertrophic cardiomyopathy. Hypertrophic cardiomyopathy (HCM), the most common cause of sudden cardiac death in the young, is characterized by cardiac hypertrophy, myocyte disarray, and interstitial fibrosis.

Stroke prevention in congestive heart failure.

Atherosclerosis is a systemic disease of the vessel wall that occurs in the carotid arteries, aorta, coronary arteries and other peripheral arteries. The main components of the atherosclerotic plaques are (1) connective tissue extracellular matrix, including collagen, proteoglycans and fibronectin elastic fibers; (2) cells such as monocyte-derived macrophages, T-lymphocytes, and smooth-muscle cells; and (3) crystalline cholesterol, cholesteryl esters, and phospholipids. During the progression of the artherosclerotic processes, substantial vascular remodeling occurs, resulting in enlargement of the vessel and, subsequently, in concentric or eccentric stenotic lesions.

Among the imaging techniques to assess atherosclerotic plaques in humans, high-resolution magnetic resonance (MR) has emerged as the leading in vivo modality for plaque characterization.

The pathogenesis of left ventricular hypertrophy (LVH) or hypertensive left ventricular hypertrophy (LVH) involves mechanical, hormonal, and neural factors. The left ventricle increases its mass and wall thickness to oppose a rise in the pressure load. Left ventricular hypertrophy (LVH) is a major cardiovascular risk factor for morbidity and mortality e.g. Myocardial hypertrophy, heart failure, heart failure related to hypertension, and other cardiovascular complications independent of blood pressure (BP). The individual prognosis of LVH can be determined by echocardiography.

Nephrosclerosis or hypertensive nephrosclerosis is characterized by interstitial accumulation of the extracellular matrix components collagen types I, III, IV, V, and VI and fibronectin (remodeling). These matrix changes were accompanied by a progressive infiltration of mononuclear cells in the cortical interstitium, suggesting that the macrophages and T lymphocytes contribute to the development of fibrogenesis.

Renal arteriopathy as a consequence of hypertension. This disease leads to thickening of the arterial wall characterized by elastic disorganisation, hypertrophied smooth muscle cells and collagen bundles.

Proteinuria in chronic renal disease. Intraglomerular hypertension promotes proteinuria, which further activates the renin-angiotensin system (RAS).

Renal Hyperfiltration such as after portal renal ablation. Partial ablation of renal mass initiates a cycle of progressive glomerular injury in the remnant. This process is associated with glomerular hypertrophy, hyperfiltration and systemic hypertension.

Diabetic nephropathy is characterized by a persistent albuminuria (> 300 mg/ 24 h or 200 µg/min), which disease can be diagnosed clinically if the following additional criteria are fulfilled: presence of diabetic retinopathy and no clinical or laboratory evidence of kidney or urinary tract disease other than diabetic glomerulosclerosis. Diabetic nephropathy is the commonest cause of endstage renal failure in the Western World.

Diabetic retinopathy (DR) and diabetic macular edema (DME) are common vascular complications in patients with diabetes and may have a sudden and debilitating impact on visual acuity (VA), eventually leading to blindness. Advanced stages of DR are characterized by the growth of abnormal retinal blood vessels secondary to ischemia. These blood vessels grow in an attemptto supply oxygenated blood to the hypoxic retina. At any time during the progression of DR, patients with diabetes can also develop DME, which involves retinal thickening in the macular area. DME occurs after breakdown of the blood-retinal barrier because of leakage of dilated hyperpermeable capillaries and microaneurysms.

Diabetic neuropathy is the least understood diabetes vascular complication which results in morphological and metabolic changes in peripheral nerves.

In the present description, the term "treatment" includes both prophylactic or preventative treatment as well as curative or disease suppressive treatment, including treatment of patients at risk of contracting the disease or suspected to have contracted the disease as well as ill patients. This term further includes the treatment for the delay of progression of the disease.

The term "curative" as used herein means efficacy in treating ongoing cardiovascular diseases or damages, renal diseases or damages, Heart Failure, or Heart Failure associated diseases.

The term "prevention" means prophylactic administration of the combination to healthy patients to prevent the outbreak of the conditions mentioned herein. Moreover, the term "prevention" means prophylactic administration of such combination to patients being in a pre-stage of the conditions, to be treated.

The term "prophylactic" means the prevention of the onset or recurrence of cardiovascular diseases or damages, renal diseases or damages, Heart Failure, or Heart Failure associated diseases.

The term "delay of progression" as used herein means administration of the active compound to patients being in a pre-stage or in an early phase of the disease to be treated, in which patients for example a pre-form of the corresponding disease is diagnosed or which patients are in a condition, e.g. during a medical treatment or a condition resulting from an accident, under which it is likely that a corresponding disease will develop.

In a further embodiment, it is an objective of the present invention to administer a dose of DPP-IV inhibitor which will treat the conditions underlying HF, including, without limitation, decreased cardiac contractility, abnormal diastolic compliance, reduced stroke volume, infection of the heart valves and/or muscle itself (endocarditis and/or myocarditis) and decreased cardiac output, while minimizing, attenuating or reducing deleterious effects commonly associated with the long-term administration of other prior art compounds , such as oxygen-wasting effects, arrhythmias, and exacerbation of angina pectoris. Preferably, the dose of DPP-IV inhibitor is such that deleterious effects commonly associated with the long-term administration of other prior art compounds are eliminated.

Most preferably, the HF condition to be treated is secondary to either idiopathic dilated cardiomyopathy (IDCM) and/or coronary ischemic disease (coronary artery disease--CAD).

Most preferably, the HF condition is Heart Failure associated with diabetes e.g. type 2 diabetes or Heart Failure associated with hypertension.

Most preferably the invention relates to the treatment or prevention of Heart Failure or heart failure associated diseases, in human subjects with diabetes, type 2 diabetes or hypertension.

A further aspect of the present invention is the use of a pharmaceutical composition comprising as active ingredients a DPP-IV, in free form or in form of a pharmaceutically acceptable salt thereof for the preparation of a pharmaceutical composition for the prevention, delay of progression or treatment of cardiovascular diseases or damages, renal diseases or damages, Heart Failure, or Heart Failure associated diseases.

The invention also relates to a pharmaceutical composition comprising, as active ingredients a DPP-IV inhibitor, in free form or in form of a pharmaceutically acceptable salt thereof.

Another aspect of the present invention is the use of a pharmaceutical composition comprising, as active ingredients a DPP-IV, in free form or in form of a pharmaceutically acceptable salt thereof for the preparation of a pharmaceutical composition for the prevention, delay of progression or treatment of cardiovascular diseases or damages, renal diseases or damages, Heart Failure, or Heart Failure associated diseases.

The invention also relates to a method for the prevention, delay of progression or treatment of cardiovascular diseases or damages, renal diseases or damages, Heart Failure, or Heart Failure associated diseases, comprising administering to a warm-blooded animal, including man, in need thereof jointly therapeutically effective amounts of a pharmaceutical composition comprising, as active ingredients a DPP-IV inhibitor, in free form or in form of a pharmaceutically acceptable salt thereof.

The pharmaceutical compositions according to the invention can be prepared in a manner known per se and are those suitable for enteral, such as oral or rectal, and parenteral administration to mammals (warm-blooded animals), including man, comprising a therapeutically effective amount of the pharmacologically active compound, alone or in combination with one or more pharmaceutically acceptable carries, especially suitable for enteral or parenteral application.

These pharmaceutical preparations are for enteral, such as oral, and also rectal or parenteral, administration to homeotherms, with the preparations comprising the pharmacological active compound either alone or together with customary pharmaceutical auxiliary substances. For example, the pharmaceutical preparations consist of from about 0.1 % to 90 %, preferably of from about 1 % to about 80 %, of the active compound. Pharmaceutical preparations for enteral or parenteral, and also for ocular, administration are, for example, in unit dose forms, such as coated tablets, tablets, capsules or suppositories and also ampoules. These are prepared in a manner that is known per se, for example using conventional mixing, granulation, coating, solubulizing or lyophilising processes. Thus, pharmaceutical preparations for oral use can be obtained by combining the active compound with solid excipients, if desired granulating a mixture which has been obtained, and, if required or necessary, processing the mixture or granulate into tablets or coated tablet cores after having added suitable auxiliary substances.

The dosage of the active compound can depend on a variety of factors, such as mode of administration, homeothermic species, age and/or individual condition.

In the field of heart failure disease, the preferred patient population age is from 50 years onwards, most preferred from 65 years onwards.

Preferred patients for the uses or methods according to the present invention are patients or animals suffering from hypertension or diabetes.

Preferred dosages, for those active ingredients of the pharmaceutical combination according to the present invention that are commercially available, are especially therapeutically effective commercially available dosages.

The dosage of the active compound can depend on a variety of factors, such as mode of administration, homeothermic species, age and/or individual condition.

The corresponding active ingredient or a pharmaceutically acceptable salt thereof may also be used in form of a hydrate or include other solvents used for crystallization.

For these indications, the exact dosage will of course vary depending upon the compound employed, mode of administration and treatment desired. The compound may be administered by any conventional route, non-oral or preferably orally.

In general, satisfactory results are obtained when administered at a daily dosage of from about 0.01 to 100mg/kg, more preferred doses ranged from 0.1 to 50mg/kg For the larger mammals, an indicated total daily dosage is in the range from about 0.01 to 100mg/kg of the compound, conveniently administered in divided doses 2 to 4 times a day in unit dosage form containing for example from about 10 to about 100 mg of the compound in sustained release form.

Another preferred daily oral dosage is between 1 and 500 mg preferably between 10 and 100 mg e.g. 10 mg, most preferably between 25 and 100 mg or between 25 and 50, e.g. 25 mg or 40 or 50 or 70 or 100 mg.

Appropriate unit doses for oral administration contain for example about 10 to about 100 mg of the compounds. Appropriate doses for parenteral administration contain for example about 10 to about 50 mg or 25 to about 100 mg of the compound, e.g. 25 mg, 50 mg, 75 mg or 100 mg.

The compounds may be administered in similar manner to known standards for uses in these utilities. The suitable daily dosage for a particular compound will depend on a number of factors such as its relative potency of activity. A person skilled in the pertinent art is fully enabled to determine the therapeutically effective dosage.

The compound of the invention may be administered in free base for or as a pharmaceutically acceptable acid addition or quaternary ammonium salt. Such salts may be prepared in conventional manner and exhibit the same order of activity as the free forms.

If these compounds have, for example, at least one basic center, they can form acid addition salts. Corresponding acid addition salts can also be formed having, if desired, an additionally present basic center. The compounds having an acid group (for example COOH) can also form salts with bases. For example, the compounds to be combined can be present as a sodium salt, as a maleate or as a dihydrochloride. The active ingredient or a pharmaceutically acceptable salt thereof may also be used in form of a hydrate or include other solvents used for crystallization.

The pharmaceutical compositions according to the invention can be prepared in a manner known per se and are those suitable for enteral, such as oral or rectal, and parenteral administration to mammals (warm-blooded animals), including man, comprising a therapeutically effective amount of the pharmacologically active compound, alone or in combination with one or more pharmaceutically acceptable carries, especially suitable for enteral or parenteral application.

Furthermore, the applicant has discovered a particular regimen improving the treatment of cardiovascular diseases or damages, renal diseases or damages, Heart Failure, or Heart Failure associated diseases. Surprisingly, if the DPP-IV inhibitor is taken in connection with the meal, preferably shortly before or at the beginning of the meal, optionally during the meal or even shortly after. The regimen meal-related according to the present invention results in an unexpected lowering of the cardiovascular diseases or damages, renal diseases or damages, Heart Failure, or Heart Failure associated diseases particularly in patients with diabetes e.g. type 2 diabetes or hypertension.

The meal-related regimen according to the present invention particularly results in an unexpected lowering of atherosclerosis in patients with diabetes particularly with type 2 diabetes.

Thus in a further aspect, the present invention relates to the use of a DPP-IV inhibitor preferably (S)-1-[(3-hydroxy-1-adamantyl) amino] acetyl-2-cyano-pyrrolidine (LAF237) of formula (I) or pharmaceutically acceptable salt thereof, for the manufacture of a medicament for the prevention, delay of progression or the treatment of cardiovascular diseases or damages, renal diseases or damages, Heart Failure, or Heart Failure associated diseases, wherein the DPP-IV inhibitor is to be administered in relation to meals.

The present invention relates furthermore to a method for the prevention, delay of progression or treatment of cardiovascular diseases or damages, renal diseases or damages, Heart Failure, or Heart Failure associated diseases comprising administering to a warm-blooded animal, including man, in need thereof, a therapeutically effective amount of a DPP-IV inhibitor preferably (S)-1-[(3-hydroxy-1-adamantyl) amino] acetyl-2-cyano-pyrrolidine (LAF237) of formula (I), the administration of the DPP-IV inhibitor being in relation to meals.

Use or method as described above for the treatment of a patient with diabetes particularly with type 2 diabetes, most preferably for the treatment or prevention of atherosclerosis.

Use or method as described above for the treatment of a patient with diabetes particularly with type 2 diabetes or hypertension, most preferably for the treatment or prevention of Heart Failure, or Heart Failure associated diseases.

Use or method as described above for the treatment of a patient with diabetes particularly with type 2 diabetes, most preferably for the treatment or prevention of diabetic macular edema, diabetic neuropathy, diabetic retinopathy, myocardial infarction, endothelial dysfunction with pro-inflammatory and pro-oxidant states, endothelial dysfunction, restenosis, nephropathy, stroke, diabetic nephropathy or coronary heart disease.

The designation "meal" as used in the present text is intended to mean breakfast, lunch dinner or midnight snack.

When the expression "meal-related" is used in the present text in connection with the administration of a DPP-IV inhibitor it preferably designates that the DPP-IV inhibitor is administered shortly before or at the beginning of the meal. However, the administration can obviously also take place during the meal or even shortly after without deviating from the idea behind the invention. Thus, the expression "meal-related" preferably means from about 30 preferably 10 minutes before the meal starts to about 10 minutes after the meal is finished, more preferred from about 5 minutes before the meal starts until the meal is finished, most preferred at the beginning of the meal.

The pharmaceutical activities as effected by administration of representatives of the class of DPP-IV inhibitors used according to the present invention can be demonstrated e.g. by using corresponding pharmacological models known in the pertinent art. The person skilled in the pertinent art is fully enabled to select a relevant animal test model to prove the herein before and hereinafter indicated therapeutic indications and beneficial effects.

Experimental protocols, which are by no way limitative, to prove pharmacological activity of the compounds and composition according to the invention in cardiovascular or renal diseases, are described below (see also former pages);
➢ The evaluation of the cardiovascular benefic effects especially in diabetes of the DPP-IV inhibitors can be performed using models such as the Zucker fatty rat as described in the publication of Nawano et al., Metabolism 48: 1248-1255, 1999. Also, studies using diabetic spontaneously hypertensive rats are described in the publication of Sato et al., Metabolism 45:457-462, 1996. Furthermore, rat models such as the Cohen-Rosenthal diabetic hypertensive rat (Rosenthal et al., Hypertension. 1997;29:1260-1264) may also be used for the simultaneous assessments of the effects of the DPP-IV inhibitor on blood pressure and cardiovascular diseases.
➢ The evaluation of the cardiovascular benefic effects especially in Left ventricular hypertrophy can be assessed as described by Gaudio C. and col. "Comparative effects of irbesartan versus amlodipine on left ventricular mass index in hypertensive patients with left ventricular hypertrophy "J Cardiovasc Pharmacol. 2003 Nov;42(5):622-8.
➢ The evaluation of the benefic effects on atherosclerosis of the DPP-IV inhibitors can be performed using protocols such as described in the publication of Prescott MF and col. "Effect of matrix metalloproteinase inhibition on progression of atherosclerosis and aneurysm in LDL receptor-deficient mice overexpressing MMP-3, MMP-12, and MMP-13 and on restenosis in rats after balloon injury"; Ann N Y Acad Sci. 1999 Jun 30;878:179-90.
➢ The evaluation of the benefic effects on Left ventricular hypertrophy of the DPP-IV inhibitors can be performed using protocols such as described in the publication of Arnfried U and col. "A meta-analysis of the effects of treatment on left ventricular mass in essential hypertension"; Am J Med. 2003 Jul;115(1):41-6.

Experimental protocols, which are by no way limitative, to prove pharmacological activity of the compounds and composition according to the invention in heart failure and heart failure associated diseases, are described below (see also former pages);
➢ For the evaluation that the DPP-IV inhibitors according to the present invention may be used for the treatment of heart failure, for example, the methods as disclosed by Smith HJ, Nuttall A: Experimental models of heart failure. Cardiovasc Res 1985, 19, 181-186 may be applied. Molecular approaches such as transgenic methods are also described, for example by Luft et al.: Hypertension-induced end-organ damage. "A new transgernic approach for an old problem." Hypertension 1999, 33, 212-218. Also, rat models of hypertension and cardiac failure as described by Doggrell SA and Brown L (Cardiovasc Res 1998, 39: 89-105) may be used for the pharmacological evaluation of the DPP-IV inhibitors. The evaluation of the cardiovascular benefic effects especially in diabetes of the agents given alone or in combination can be performed using models such as the Zucker fatty rat as described in the publication of Nawano et al., Metabolism 48: 1248-1255, 1999.
➢ Additional experimental protocols are described in the patents US 6,087,360 (using farm pigs as heart failure model) and US 20030045469.
➢ A useful *Heart Failure* clinical trial is described by Stephan Schmidt-Schweda and Christian Holubarsch (First clinical trial with etomoxir in patients with chronic congestive heart failure; Clinical Science (2000) 99, 27-35).
➢ Other clinical trials are described by Jay N. Cohn, M.D. and Gianni Tognoni, M.D. (Volume 345:1667-1675, December 6, 2001 - Number 23 A Randomized Trial of the Angiotensin-Receptor Blocker Valsartan in Chronic Heart Failure) and the patent US 5,998,458 (example 1).

The structure of the active agents identified by code nos., generic or trade names may be taken from the actual edition of the standard compendium "The Merck Index" or from databases, e.g. Patents International (e.g. IMS World Publications). The corresponding content thereof is hereby incorporated by reference. Any person skilled in the art is fully enabled to identify the active agents and, based on these references, likewise enabled to manufacture and test the pharmaceutical indications and properties in standard test models, both *in vitro* and *in vivo.*

The pharmacological activity may, for example, be demonstrated in a clinical study or in the test procedure as essentially described hereinafter in a manner known to the skilled person.

Preferred DPP-IV inhibitors for the uses and methods of the present invention are 1-{2-[(5-cyanopyridin-2-yl) amino] ethylamino} acetyl-2 (S)- cyano-pyrrolidine dihydrochloride (DPP728), especially the dihydrochloride thereof, (S)-1-[(3-hydroxy-1-adamantyl)amino]acetyl-2-cyano-pyrrolidine (LAF237), and L-threo-isoleucyl thiazolidine (compound code according to Probiodrug: P32/98 as described above), MK-0431, GSK23A, BMS-477118, 3-(aminomethyl)-2-isobuthyl-1-oxo-4-phenyl-1,2-dihydro-6-isoquinolinecarboxamide and 2-{[3-(aminomethyl)-2-isobuthyl-4-phenyl-1-oxo-1,2-dihydro-6-isoquinolyl]oxy}acetamide and optionally in any case pharmaceutical salts thereof.

### EXPERIMENTAL PART

The following examples can be carried out with DPP-IV inhibitors to show their claimed activity.

### Example 1:

Animals: Forty-eight, 8 week old, male Sprague Dawley rats are anaesthetized with enflurane (Abbott Australasia, Kumal, NSW, Australia) and a osmotic minipump (Alzet Model 2002, Alzet Corporation, Palo Alto) inserted in the interscapular region. Rats are randomized to receive minipumps filled vehicle (0.15 M NaCl, 1 mmol/l acetic acid) either with or without angiotensin II (58 ng/min), as previously described (Cao Z et col. Kidney Int 58:2437-2451, 2000). Animals are then further randomized to receive either no treatment or DPP-IV inhibitors at a dose of 60 mg/kg by daily gavage and sacrificed 12 days later. Rats have unrestricted access to water and standard rat chow. Systolic blood pressure (SBP) is measured by indirect tail-cuff plethysmography in prewarmed unanesthetized animals as previously described (Bunag RD; Journal of Applied Physiology 34:279-282, 1973)*.* In each group of 16 animals, 8 are used for histological studies and eight are used for gene expression analyses. In the latter group, animals are sacrificed by decapitation after which the mesenteric vessels are removed and stripped of surrounding fat, connective tissue and veins to yield the superior mesenteric arterial tree as previously described (Rumble J et al.; J Hypertension 14:601-607, 1996). The vessels are weighed, snap frozen in liquid nitrogen and subsequently stored at -80°C.

Histochemistry and immunohistochemistry: Histological studies of vascular architecture are performed in a subset of animals (n = 8/group). Animals in this subgroup are anesthetized with pentobarbital sodium (Nembutal, Bomac Laboratories, Asquith, Australia) and vessels are perfused at arterial pressure with 10% neutral buffered formalin via an intra-aortic cannula. Tissues are then prepared as previously described (Cooper ME et col.; Diabetes 43:1221-1228, 1994). In brief, mesenteric vessels are placed in ice cold phosphate buffer where fat, connective tissue and veins are removed by blunt dissection. The resultant vessel preparation are then embedded in paraffin. Sections are then either histochemically or immunohistochemically stained. Histochemical staining comprised the use of either haematoxlyin and eosin or Masson's trichrome (Masson P; J Tech Methods 2:75-90, 1929) to examine extracellular matrix. Smooth muscle is immunostained using a polyclonal α-smooth muscle actin antibody (Biogenes, San Ramon, CA) and immunohistochemistry is performed using the indirect avidin-biotin complex method as previously described (Rumble JR et cal.; J Clin Invest 99:1016-1027, 1997).

Quantification of histopathology: The proportion of vessel wall occupied by the media is identified by immunolabelling for α-smooth muscle actinand the ratio of the wall:lumen quantified using of a video-imaging system (Video Pro 32, Leading Edge, Bedford Park, South Australia, Australia), connected to a light microscope (Zeiss, Oberkocken, Germany) with photographic attachment (Axiophot, Zeiss, Oberkocken, Germany). Using this device, the media (areas stained with the anti-actin antibody) and corresponding lumenal areas are determined in a median of 20 (range 10-35) vessels per animal and expressed as wall:lumen ratio, as previously described in other models of vascular hypertrophy (Kakinuma Yet col.; Kidney Int 42:46-55., 1992).

Quantification of ECM is performed on trichrome-stained sections by calculating the proportion of area occupied by ECM using computer-assisted image analysis as previously described (Lehr HA, et col. J Histochem Cytochem 45:1559-1565, 1997). In brief, the color ranges for matrix (blue on trichrome stained sections), media smooth muscle (red on trichrome stained sections) are selected and image analysis is performed using a chromogen-separating technique (Lehr HA, et col.). The entire section is examined by light microscopy (Olympus BX-50, Olympus Optical, Tokyo, Japan) and digitized using a high resolution camera (Fujix HC-2000, Fujifilm, Tokyo, Japan). All images are obtained using a 20X objective lens. Digitized images are then captured on a Power Macintosh G3 computer (Apple Computer Inc., Cupertino, CA) equipped with an in-built graphic board and evaluated using analytical software (Analytical Imaging Software, Ontario).

Statistics: All data are shown as mean ± SE unless otherwise specified. Data are analyzed by analysis of variance (ANOVA) using the StatView IV program (Brainpower, Calabasas, CA) on a Macintosh G3. Comparisons between group means are performed by Fisher's least significant difference method. A p value of less than 0.05 is considered statistically significant.

### Results:

Animal Data: Angiotensin II infusion is associated with increased systolic blood pressure (SBP) compared with vehicle treated animals (141 ± 2 mmHg versus 202 ± 7 mmHg, vehicle versus angiotensin II, p < 0.001). Mesenteric vessel weight is also increased in angiotensin II-infused rats (52 mg, p < 0.001), compared with control animals receiving vehicle (32 mg, p < 0.001). DPP-IV inhibitors have no significant effect on systolic blood pressure (205 ± 10 mmHg) but significantly reduce mesenteric weight in animals receiving angiotensin II (42 mg, p < 0.001).

Histology and immunohistochemistry: Histomorphometric analysis reveal a significant increase in the media wall:lumen ratio in angiotensin II-infused rats (Vehicle -> 0.39; angiotensin II-infused rats -> 0.52 , p < 0.001). Treatment with a DPP-IV inhibitor can e.g. significantly reduce the wall:lumen ratio in both groups to levels approaching those in control animals. Trichrome stained sections demonstrate both media hypertrophy and expansion of collagenous ECM in the mesenteric vessels of animals receiving angiotensin II-infusion. This experience shows that DPP-IV inhibitor treatment can reduce both media hypertrophy and the extent of ECM expansion in angiotensin II-infused.

Example 2: Nephropathy: Antiproteinuric action in rats with non-insulin-dependent (NIDD) diabetes (Wistar fatty rats) [protocol described by H. Ikeda et al., Diabetes 30, 1045 (1981) or by focal glomerulosclerosis model; T.W. Meyer and H.G. Renake, Am. J. Physiol. 254, F856 (1988) or in WO 01/64200]

This unforeseeable range of properties means that DPP-IV inhibitors is of particular interest for the manufacture of a medicament for the treatment of the herein described diseases especially hypertrophy or hypertrophic remodeling in the cardiovascular system and or in the kidney, and the associated diseases described herein.

This effect can especially be clinically relevant for patients with injuries in relation to hypertension such as described herein.

### Experiment 3: Effect on Cardiac Hypertrophy

Materials and Methods: Animal-housed male Wistar rats weighing 100-120 g are used: 5 per cage (cage size: 425 mm×266 mmx180 mm with a sawdust litter) at a temperature of 21±10° C. and 50±15% humidity with a 12/12 h light/darkness cycle and with 15-20 air changes/hour. The animals are fed with LP ALTROMIN (REIPER) feed and spring water ad libitum.

Induction of Cardiac Hypertrophy: Left ventricular hypertrophy is induced in rats anaesthetised with Nembutal sodium, by means of constriction of the abdominal aorta with a clip (00.8 mm) placed in the abdominal aorta between the diaphragm and the renal branches; one group of animals, which is then used as a control group, underwent the same operation but is not submitted to constriction of the aorta (shams).

The animals are thus randomly assigned to the following groups:
Shams: operated on without aortic constriction (n=8)
Controls: operated on with aortic constriction (n=8)
CLO: operated on with aortic constriction and to be treated for 12 weeks, from the day after the operation with DPP-IV inhibitors in the feed (60-100 mg/kg/day (n=11).
Evaluation of Cardiac Function: At the end of treatment, cardiac function is evaluated, in the animals anaesthetised with Nembutal sodium, by means of a polyethylene catheter inserted in the left ventricle via the carotid and connected up to a pressure transducer (Statham p23 XL) and to an amplifier (Biomedica, Mangoni bm 61).

The parameters to be recorded are: heart rate, ventricular systolic and end- diastolic pressure, and the positive and negative derivatives of ventricular pressure, which are recorded on a personal computer by means of a special data acquisition system (IDAS). The measurements are taken for a period of 30 minutes.

Macroscopic Assessment: At the end of the experiments the animals are sacrificed by means of a lethal dose of Nembutal, the abdominal cavity was opened, the organs are exteriorised in order to verify correct positioning of the aortic clip, and the heart, lungs and liver are then removed and carefully dried and weighed after macroscopic observation of any abnormalities.

Statistical Analysis: The data are expressed as mean ± standard deviation and are compared using Student's t-test for independent data. Differences with P<0.05 were regarded as statistically significant.

### Results

Weight Parameters: The weight parameters measure at the end of the experiment is evaluated; the body weight of the animals does not change significantly either as a result of constriction of the aorta or as a result of the treatments. Aortic constriction induces a significant ventricular hypertrophy; in fact, the heart weight of the animals with the clip increases by approximately 35% as compared to the shams (P<0. 05); the treatments administered do not modify heart weight as compared to the untreated controls. Liver and lung weights are not changed either as a result of aortic constriction or as a result of the treatments administered.

Cardiac Function : Aortic constriction induces a significant increase both in left ventricular systolic pressure and in end-diastolic pressure; the pressure developed, and the positive and negative derivatives of ventricular pressure show no statistically significant changes in absolute values in the animals with aortic constriction as compared to the shams.

If the pressure developed is normalised for ventricular weight, a statistically significant reduction in this parameter can be observed in the animals with aortic constriction; similarly, the positive derivative of ventricular pressure can significantly be reduced if it is normalised for ventricular systolic pressure.

Treatment of the animals with aortic constriction normalises end-diastolic pressure, and the pressure developed in relation to ventricular weight, with a significant improvement in both the positive and negative derivatives of ventricular pressure.

Treatment with DPP-IV inhibitors can induce e.g. a further increase in left ventricular systolic pressure and normalise end-diastolic pressure in the animals with aortic constriction.

The pressure developed in relation to ventricular weight, and both the positive and negative derivatives of ventricular pressure can significantly be improved by treatment with DPP-IV inhibitors.

### Experiment 4. Effect on endothelial function (Heart Failure)

This example describes a study to be conducted to determine the effect of the treatment of the invention on endothelial function in Watanabe rabbits, known to develop complex atherosclerotic lesions during the first year of life. As previously mentioned, endothelial dysfunction is linked to the pathophysiology of HF.

The rabbits enter the study at 7 to 8 months of age, and are randomized into three groups, a first group to be sacrificed immediately for baseline measurements, a second group (n=10) which receives a DPP-IV inhibitor, and a third group (n=10) which receives sham treatments comprising placebo (control animals).

The treatment comprise a daily administration of DPP-IV inhibitor over a period of 10 weeks.

All animals are sacrificed at 11 months of age. Ring preparations are taken from the iliac arteries of the animals and are evaluated for the amount of relaxation induced by acetylcholine (an endothelial-dependent vasodilator) after being treated with phenylephrine (a vasoconstrictor).

Evaluation of the ring preparations e.g. show a significant increase in endothelial-mediated vasorelaxation in the treated animals as compared to the control animals.

No relaxation is observed when the endothelium is removed from the ring preparations, further confirming the endothelium-specific effect of the treatment of the invention. DPP-IV inhibitors are able to restore the attenuated endothelium-dependent relaxation

The invention has been described above by reference to preferred embodiments but, as those skilled in the art will appreciate, many additions, omissions and modifications are possible all within the scope of the claims below.

All patents and literature references cited in this specification are hereby incorporated by reference in their entirety. In case of inconsistencies, the present description, including the definitions and interpretations, will prevail.

## Claims

1. A method for the prevention, delay of progression or treatment of cardiovascular diseases or damages, renal diseases or damages, Heart Failure, or Heart Failure associated diseases, comprising administering to a warm-blooded animal, including man, in need thereof, a therapeutically effective amount of a DPP-IV inhibitor.

2. The method according to claim 1 wherein the DPP-IV inhibitor is selected from 1-{2-[(5-cyanopyridin-2-yl) amino] ethylamino} acetyl-2 (S)-cyano-pyrrolidine dihydrochloride, (S)-1-[(3-hydroxy-1-adamantyl)amino]acetyl-2- cyano-pyrrolidine, L-threo-isoleucyl thiazolidine (P32/98), MK-0431, GSK23A, BMS-477118, 3-(aminomethyl)-2-isobuthyl-1-oxo-4-phenyl-1,2-dihydro-6-isoquinolinecarboxamide and 2-{[3-(aminomethyl)-2-isobuthyl-4-phenyl-1-oxo-1,2-dihydro-6-isoquinolyl]oxy}acetamide or, in each case , a pharmaceutically acceptable salt thereof.

3. The method of claim 1 wherein the DPP-IV inhibitor is (S)-1-[(3-hydroxy-1-adamantyl) amino] acetyl-2-cyano-pyrrolidine, or a pharmaceutically acceptable salt thereof.

4. Use of a DPP-IV inhibitor or pharmaceutically acceptable salt thereof, for the manufacture of a medicament for the prevention, delay of progression or the treatment of cardiovascular diseases or damages, renal diseases or damages, Heart Failure, or Heart Failure associated diseases.

5. Use of a DPP-IV inhibitor or pharmaceutically acceptable salt thereof, for the manufacture of a medicament for the prevention, delay of progression or the treatment of cardiovascular diseases or damages, renal diseases or damages, Heart Failure, or Heart Failure associated diseases, wherein the DPP-IV inhibitor is to be administered in relation to meals.

6. Use according to claim 4 or 5, wherein the DPP-IV inhibitor is selected from 1-{2-[(5-cyanopyridin-2-yl) amino] ethylamino} acetyl-2 (S)-cyano-pyrrolidine dihydrochloride, (S)-1-[(3-hydroxy-1-adamantyl)amino]acetyl-2- cyano-pyrrolidine, L-threo-isoleucyl thiazolidine (P32/98), MK-0431, GSK23A, BMS-477118, 3-(aminomethyl)-2-isobuthyl-1-oxo-4-phenyl-1,2-dihydro-6-isoquinolinecarboxamide and 2-{[3-(aminomethyl)-2-isobuthyl-4-phenyl-1-oxo-1,2-dihydro-6-isoquinolyl]oxy}acetamide or, in each case , a pharmaceutically acceptable salt thereof.

7. Use according to claim 4 or claim 5, wherein the DPP-IV inhibitor is (S)-1-[(3-hydroxy-1-adamantyl) amino] acetyl-2-cyano-pyrrolidine, or pharmaceutically acceptable salt thereof.

8. Use according to any one of the claims 4 to 7, or method according to any one of the claims 1 to 3, wherein the cardiovascular diseases or damages are selected from cardiac hypertrophy, cardiac remodeling after myocardial infarction, pulmonary congestion and cardiac fibrosis in dilated or in hypertrophic cardiomyopathy, cardiomyopathy such as dilated cardiomyopathy or hypertrophic cardiomyopathy or diabetic cardiomyopathy, left or right ventricular hypertrophy, diabetic myopathy, stroke prevention in congestive heart failure, hypertrophic medial thickening in arteries and/or in large vessels, mesenteric vasculature hypertrophy, or artherosclerosis.

9. Use according to any one of the claims 4 to 7, or method according to any one of the claims 1 to 3, wherein the renal diseases or damages are selected from renal hyperfiltration such as after portal renal ablation, proteinuria in chronic renal disease, renal arteriopathy as a consequence of hypertension, nephrosclerosis, hypertensive nephrosclerosis or mesanglial hypertrophy.

10. Use according to any one of the claims 4 to 7, or method according to any one of the claims 1 to 3, wherein the cardiovascular and renal diseases or damages are selected from cardiac hypertrophy such as left ventricular hypertrophy, pulmonary congestion or cardiomyopathy such as dilated cardiomyopathy or hypertrophic cardiomyopathy, or mesanglial hypertrophy.

11. Use according to any one of the claims 4 to 7, or method according to any one of the claims 1 to 3, wherein the vascular disease or damage is selected from hypertrophic medial thickening in arteries and/or in large vessels, mesenteric vasculature hypertrophy or artherosclerosis, in an animal or human suffering from hypertension or diabetes.

12. Use or method according to claim 11 wherein the vascular disease or damage is atherosclerosis.

13. Use according to any one of the claims 4 to 7, or method according to any one of the claims 1 to 3, to improve left ventricle function.

14. Use according to any one of the claims 4 to 7, or method according to any one of the claims 1 to 3, for treatment of Heart Failure, or Heart Failure associated diseases.

15. Use according to any one of the claims 4 to 7, or method according to any one of the claims 1 to 3, wherein the Heart Failure condition to be treated is secondary to either idiopathic dilated cardiomyopathy and/or coronary ischemic disease.

16. Use or method according to any one of the previous claims, wherein the treated patient or animal suffers from hypertension or diabetes.

17. Use or method according to any one of the previous claims, wherein the daily oral dosage of active ingredient preferably (S)-1-[(3-hydroxy-1-adamantyl) amino] acetyl-2-cyano-pyrrolidine is between 1 and 100 mg preferably between 25 and 100 mg.

18. A pharmaceutical composition comprising a therapeutically effective amount of a DPP-IV inhibitor in combination with one or more pharmaceutically acceptable carriers for the treatment of cardiovascular diseases or damages, renal diseases or damages, Heart Failure, or Heart Failure associated diseases.
